# DEMANDE DE BREVET EUROPEEN

(11) **EP 1 421 937 A1**
(43) Date de publication de la demande: **26.05.2004**
(21) Numéro de dépôt: 03291523.3
(22) Date de dépôt: 23.08.2000
(51) Int. Cl.: A61K 31/198, A61P 5/50, A61K 31/195

(54) **Utilisation d'acides amines pour la fabrication de medicaments destinés au traitement des insulino-resistances**

(30) Priorité: 27.08.1999 FR 9910874
(62) Demande divisionnaire de: 00958726.2
(71) Demandeur: Innodia, 34060 Montpellier (FR)
(72) Inventeur: Ribes, Gérard, 34070 Montpellier (FR); Taouis, Mohammed, 37200 Tours (FR); Petit, Pierre-Roger, 34000 Montpellier (FR); Broca, Christophe, 34000 Montpellier (FR); Sauvaire, Yves, 34095 Montferrier sur lez (FR); Pau, Bernard, 34080 Montpellier (FR)
(74) Mandataire: Peaucelle, Chantal

(57) **Abrégé**

La présente invention est relative à l'utilisation d'acides aminés, et en particulier d'un acide aminé mono- ou polyhydroxylé, et/ou de ses formes lactoniques, pour la fabrication de médicaments destinés au traitement des insulino-résistances.

## Description

La présente demande est relative à l'utilisation d'acides aminés pour la fabrication de médicaments à effets insulino-mimétiques et/ou insulino-sensibilisateurs sur les tissus périphériques cibles de l'insuline, et plus particulièrement à l'utilisation d'acides aminés pour la fabrication de médicaments destinés au traitement et à la prévention des insulino-résistances.

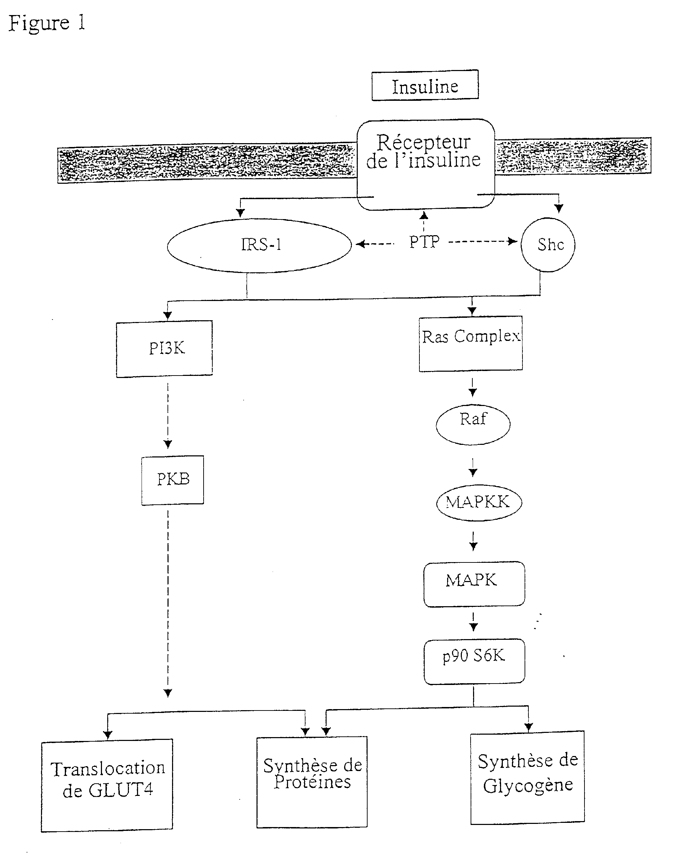

Ces dernières années, des progrès considérables ont été réalisés dans la compréhension des mécanismes moléculaires de l'action de l'insuline. Un schéma montrant les voies principales de transduction du signal insulinique est donné sur la figure 1. Le récepteur de l'insuline est un récepteur transmembranaire doué d'une activité tyrosine kinase intrinsèque (Combettes-Souverain M. et Issad T., Diab. Metab., 24, 477, 1998). La liaison de l'insuline à son récepteur entraîne l'autophosphorylation du récepteur sur ses résidus tyrosine, ce qui stimule son activité tyrosine kinase envers un certain nombre de substrats intracellulaires tels que Insulin Recepteur Substrate 1 (IRS-1), Insulin Recepteur Substrate 2 (IRS - 2) et Src homology collagen protein ou Shc. Cette activité tyrosine kinase joue un rôle déterminant dans la transmission du signal insulinique, et elle est altérée dans de nombreuses situations de résistance à l'insuline (Ricort J.M. et coll., Diabetologia, 38, 1148, 1995). Globalement les mécanismes cellulaires de l'insulino-résistance peuvent se situer au niveau de la liaison de l'hormone à son récepteur ou à une étape plus distale de post-liaison au récepteur. Comme le montre le schéma de la figure 1, la phosphorylation sur les tyrosines des IRS et de Shc par le récepteur à l'insuline permet l'activation de deux grandes voies de signalisation cellulaire, la voie des MAP kinases (MAPK) et la voie de la phosphatidylinositol 3-kinase (PI 3-K). La phosphotyrosine phosphatase (PTP) participe à la régulation de ces voies.

Les inventeurs ont précédemment rapporté l'effet insulino-sécréteur qui peut être exercé, au niveau des cellules pancréatiques, par des acides aminés hydroxylés extraits de graines de fenugrec *(Trigonella foenum graecum L.),* et en particulier par la 4-hydroxyisoleucine (4-OH-Ile) et/ou la lactone correspondante (brevets FR 2 695 317 et 2 745 718 et brevets correspondants sous priorité).

Or, les inventeurs ont maintemant mis en évidence que de tels composés sont également capables d'agir au niveau des cellules cibles de l'insuline, à savoir les tissus périphériques tels que le foie et le muscle, en exerçant une action au niveau du récepteur à l'insuline et/ou de la cascade de signalisation que l'activation de ce récepteur déclenche. L'effet observé au niveau de ces tissus périphériques correspond globalement à un effet insulino-mimétique ou insulino-sensibilisateur.

Les travaux réalisés, comme illustré par les exemples, ont en effet montré l'effet de ces composés au niveau de la cascade des phosphorylations mise en jeu sous le récepteur, notamment l'augmentation de la phosphorylation des principales protéines impliquées dans la transmission du signal insulinique. L'activité PI 3-kinase (Phosphatidylinositol 3-kinase), enzyme activée par IRS-1 phosphorylée et jouant un rôle primordial dans la translocation du transporteur de glucose GLUT 4, est également augmentée sous l'effet de tels composés. Dans les mêmes conditions d'utilisation de ces composés, l'activité phosphatase associée à la voie de signalisation du récepteur de l'insuline (activité PTP) est abaissée, ce qui est cohérent avec l'augmentation des phosphorylations évoquée ci-dessus. Ces composés sont donc capables d'agir sur les voies de signalisation qui sont normalement enclenchées par l'insuline, que cela soit par activation des kinases, et/ou inhibition des phosphatases.

La présente demande a pour objet de tirer profit des résultats obtenus, et vise ainsi toute utilisation de tels acides aminés ou de leurs dérivés, à titre d'agent insulino-mimétique ou insulino-sensibilisateur. Elle vise notamment toute utilisation de tels composés pour la fabrication de médicaments à effets insulino-mimétiques et/ou insulino-sensibilisateurs. Ces effets peuvent être observés au niveau des tissus périphériques cibles de l'insuline. Comme ces composés agissent sur les voies de signalisation qui sont normalement enclenchées par l'insuline, ils peuvent en effet servir de substituts, de compléments, de potentialisateur et de sensibilisateurs à l'insuline.

La présente demande vise ainsi l'utilisation, à titre d'agent insulino-mimétique ou insulino-sensibilisateur, de tout acide aminé ou dérivé d'acide aminé qui exerce une diminution de l'activité phosphatase PTP et/ou une augmentation de l'activité PI 3-kinase, d'un niveau équivalent, voire supérieur à la diminution, ou respectivement augmentation, provoquée par l'insuline. Tout moyen permettant de constater un tel effet de diminution de l'activité PTP ou d'augmentation de l'activité PI 3-kinase est approprié. Les exemples qui suivent en donnent des illustrations.

De tels composés correspondent de manière remarquable à des acides aminés qui ne reconnaissent pas le récepteur de l'insuline sur son site de liaison, mais qui agissent au niveau post-récepteur, dans les conditions rapportées dans les exemples pour la 4-OH-Ile, et correspondent également aux dérivés de ces acides aminés qui ont conservé, tout au moins en nature, les propriétés de non-reconnaissance du récepteur de l'insuline sur son site de liaison et d'activité au niveau post-récepteur que présente l'acide aminé parent dudit dérivé.

De manière avantageuse, l'utilisation selon l'invention est caractérisée en ce qu'elle porte sur un composé choisi parmi le groupe constitué par les acides aminés mono-hydroxylés, les acides aminés poly-hydroxylés, et les formes lactoniques de ces acides aminés mono- ou poly-hydroxylés.

L'invention vise tout spécialement l'utilisation de la 4-hydroxyisoleucine de formule 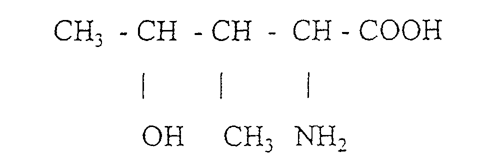 et/ou de sa forme lactonique.

En particulier, l'invention vise l'utilisation de la 4-hydroxyisoleucine (4-OH-Ile en abrégé) sous la forme de son isomère 2S, 3R, 4S, ou de la lactone correspondante.

Compte tenu des effets observés, les médicaments fabriqués conformément à l'invention sont particulièrement appropriés pour traiter les insulino-résistances, combattre ou prévenir les syndromes liés à l'insulino-résistance, et prévenir les insulino-résistances.

Or, on sait que la prise excessive de poids, la sédentarité, une alimentation mal équilibrée, le nombre de plus en plus important des personnes âgées sont des facteurs socio-économiques fréquemment rencontrés dans les pays occidentaux. Tous concourent au développement d'une insulino-résistance et d'une hyperinsulinémie compensatoire, souvent associée à l'obésité et potentiellement diabétogène. Ainsi, les acides gras libres sont parmi les premiers candidats avancés pour tenter d'expliquer l'étroite relation entre insulino-résistance, obésité et hyperinsulinisme (Mac Garry J.D., J. Cell. Biochem., 555, 29, 1994).

Cette relation prend de nos jours la dimension d'un phénomène majeur sur le plan de la santé publique. Un faisceau de preuves, à la fois cliniques et épidémiologiques, liant l'hyperinsulinémie au risque de futures maladies cardio-vasculaires, risque athérogène ou diabétogène, a permis de dresser le tableau cohérent du syndrome X de Reaven (hyperinsulinémie, insulino-résistance, augmentation des triglycérides sériques, hypertension artérielle) et des risques morbides qui lui sont associés (Reaven G.M., Diabetes, 37, 1595, 1988). De plus, selon de récentes enquêtes, l'hyperinsulinémie en favorisant la prolifération de certaines cellules épithéliales (notamment du colon) apparaît associée au risque de cancer (Hu F.B. et coll., J. Natl. Cancer Inst., 91, 542, 1999).

La présente demande vise ainsi toute utilisation desdits acides aminés et dérivés pour la fabrication d'un médicament destiné à lutter contre les insulino-résistances et les syndromes d'insulino-résistance, en particulier contre les hyperinsulinémies, l'insulino-résistance liée au vieillissement et contre des affections liées à l'obésité.

La présente demande vise également toute utilisation desdits acides aminés et dérivés pour la fabrication d'un médicament destiné à prévenir les insulino-résistances, et en particulier pour la fabrication d'un médicament destiné à diminuer le besoin en insuline exogène. De tels médicaments, du fait de leurs propriétés insulino-mimétiques et insulino-sensibilisateurs, peuvent en effet avoir pour effet de diminuer le besoin en insuline exogène que présentent les patients dont l'insuline endogène est déficitaire, voire absente comme dans le cas du diabète de type 1. Les patients atteints de diabète de type 1 souffrent en effet d'une absence totale de sécrétion d'insuline (destruction des cellules productrices) qui contraint à des apports exogènes complets d'insuline. Cette situation, outre le coût intrinsèque à de telles administrations, conduit souvent au développement d'insulino-résistances. Afin de prévenir et remédier à ces problèmes, la présente invention propose pour le traitement d'un déficit en insuline endogène, et en particulier d'une absence d'insuline endogène telle que le diabète de type 1, et pour la fabrication d'un médicament destiné à de tels traitements, d'utiliser au moins un desdits acides aminés ou dérivés. Utilisés en combinaison avec l'insuline, ils présentent l'avantage de diminuer le besoin en insuline exogène du patient (diminution des apports en insuline nécessaires), et donc de diminuer les frais de traitement tout en prévenant le développement des insulino-résistances et de leurs effets secondaires.

Les médicaments fabriqués conformément à l'invention pourront être également utilisés pour contribuer à inhiber la prolifération de certaines lignées cellulaires associées au risque d'apparition de cancers.

L'invention vise encore l'utilisation desdits dérivés, en particulier de la 4-hydroxyisoleucine et/ou de sa forme lactonique pour fabriquer des médicaments qui agissent en diminuant l'activité phosphatase associée à la voie de signalisation du récepteur-insuline, et/ou en stimulant l'activité PI 3-kinase sur IRS-1 et/ou IRS-2.

La présente demande vise également toute composition pharmaceutique, tout kit pharmaceutique, et tout médicament comprenant, de manière combinée, de l'insuline et au moins un des composés acides aminés ou dérivés définis ci-dessus. Cette combinaison peut être physique (insuline et acide aminé ou dérivé sont alors dans une même composition). Elle peut alternativement correspondre à une présentation de l'insuline d'une part, et de l'acide aminé ou dérivé d'autre part, dans des compositions physiquement distinctes, mais qui sont présentées comme associées pour une utilisation combinée (kit-of-parts). Cette utilisation combinée peut être simultanée ou différée dans le temps.

Les médicaments selon l'invention peuvent être administrés par voie orale, essentiellement, mais aussi par voie intraveineuse ou intramusculaire, et renferment des excipients qui sont choisis en fonction de la forme galénique adoptée.

La posologie sera adaptée en fonction de la pathologie à traiter.

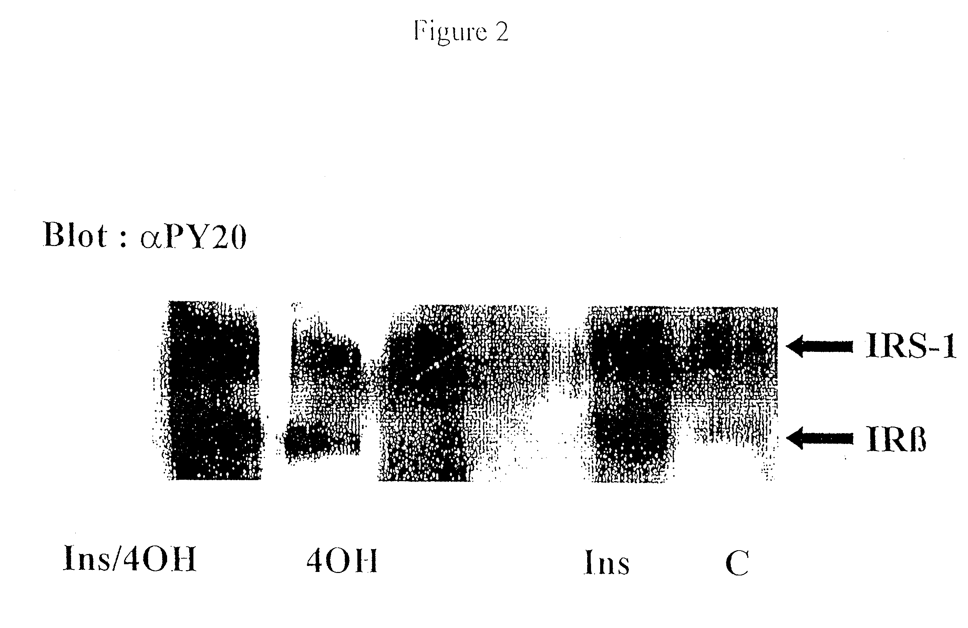

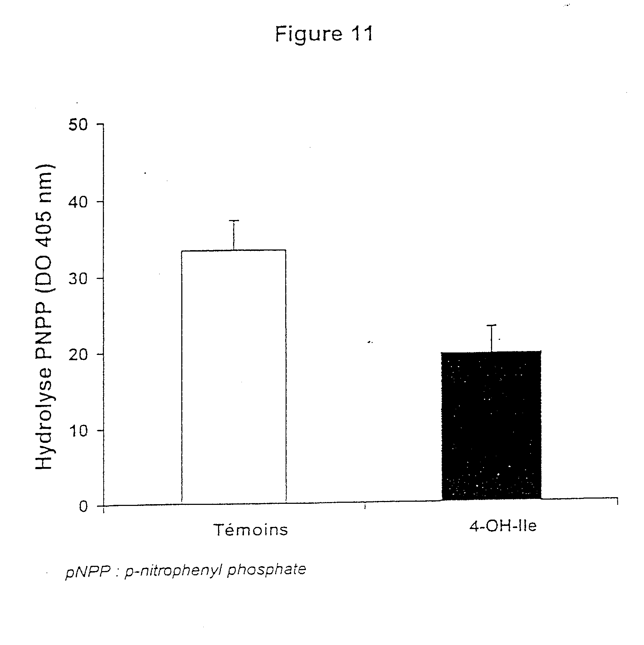

D'autres caractéristiques et avantages de l'invention sont donnés, à titre illustratif, dans les exemples qui suivent dans lesquels il est fait référence aux figures 2 à 11 (on rappelle que la figure 1 a été évoquée ci-dessus et donne le schéma des voies principales de transduction du signal insulinique), ces figures 2 à 11 représentant respectivement :
- la figure 2, l'effet de la 4-OH-Ile sur la phosphorylation du récepteur de l'insuline et de son substrat dans le foie de rat,
- la figure 3, l'effet de la 4-OH-Ile sur l'activité PI 3-kinase du foie,
- la figure 4, la liaison de l'insuline sur son récepteur dans la lignée de cellules hépatiques LMH,
- les figures 5 à 11, l'effet de la 4-OH-Ile, respectivement sur,
   . l'activité PI 3-kinase du muscle (figure 5),
   . l'insulinémie et la glycémie basales du rat (figure 6),
   . l'activité PI 3-kinase du foie (figure 7) et du muscle (figure 8) de rat diabétique de type 2,
   . l'activité PI 3-kinase du foie de rat Zucker obèse (fa/fa) (figure 9),
   . l'activité PI 3-kinase associée au récepteur PDGF ou au récepteur insuline sur le foie de rat normal (figure 10), et
   . l'activité phosphatase associée à IRS-1 au niveau du foie de rat normal (figure 11).

### Mesure de l'activité PI 3-kinase

L'activité PI 3-kinase a été mesurée sur des immunoprécipitats réalisés avec l'anticorps anti-IRS-1. Ceci a permis de déterminer l'activité enzymatique associée à l'action de l'insuline et de la comparer à l'action induite par la 4-OH-Ile.

### Protocole expérimental

Des rats normaux Wistar mâles (souche IFFA CREDO, France) ont reçu, par injection intrapéritonéale, soit de l'insuline ordinaire seule (100 U/kg) soit de la 4-OH-Ile seule (18 mg/kg), soit de l'insuline associée à la 4-OH-ILe aux mêmes posologies. Les rats témoins ont reçu par voie intrapéritonéale du chlorure de sodium à 9 ‰. Quinze minutes après l'injection, les animaux ont été sacrifiés et les tissus périphériques (foie, muscle) immédiatement prélevés et congelés dans l'azote liquide.

Pour la détermination de l'activité PI 3-kinase, les tissus sont broyés dans un tampon contenant les inhibiteurs de protéases et phosphatases ainsi qu'un agent solubilisateur (Triton), comme décrit par Taouis et coll., J. Biol. Chem., 269, 14912, 1994. Après solubilisation, les surnageants sont immunoprécipités avec l'anti-IRS- 1 et l'activité PI 3-kinase est mesurée dans l'immunoprécipitat. En effet, la réaction est initiée par l'addition d'un substrat artificiel de l'enzyme : le phosphatidylinositol (PI) et (³³P) gamma ATP. Le produit de la réaction est soumis à une chromatographie sur couche mince (TLC plate) et les niveaux de phosphorylation du PI sont mesurés par un phosphoimager STORM (Molecular Dynamics). L'activité est exprimée en unité arbitraire donnée par l'appareil (conversion de la radioactivité en luminescence grâce à un faisceau laser).

### Mesure de l'activité phosphatase associée à la voie de signalisation de l'insuline

L'activité phosphatase a été mesurée sur des immunoprécipitats réalisés avec l'anticorps anti-IRS-1 et anti-récepteur insuline.

Le protocole de solubilisation est le même que celui pour le PI 3-kinase selon la méthodologie décrite par Taouis et coll. (J. Biol. Chem., 269, 14912, 1994). Après immunoprécipitation, l'activité phosphatase a été mesurée selon la méthode rapportée par Chen. et coll. (J. Biol. Chem., 272, 8026, 1997).

### EXEMPLE 1 : Comparaison des effets de l'insuline et de la 4-OH-Ile sur la phosphorylation du récepteur-insuline et de IRS-1 dans le foie de rats normaux

Les résultats de la figure 2 montrent clairement que le traitement des animaux par une injection unique de 4-OH-Ile (200 µg/kg I.P.) induit l'activation du récepteur de l'insuline (RI) et de son substrat (IRS-1) *in vivo.* L'effet de la 4-OH-Ile (4OH est comparable à celui de l'insuline (Ins).
Ces résultats démontrent les effets insulino-mimétiques de la 4-OH-Ile dans l'activation de la phosphorylation du récepteur-insuline et de IRS-1 dont les phosphorylations sont indispensables pour l'activation de protéines effectrices telles que la PI 3-kinase.

### EXEMPLE 2 : Comparaison des effets de l'insuline et de la 4-OH-Ile sur l'activité PI 3-kinase du foie

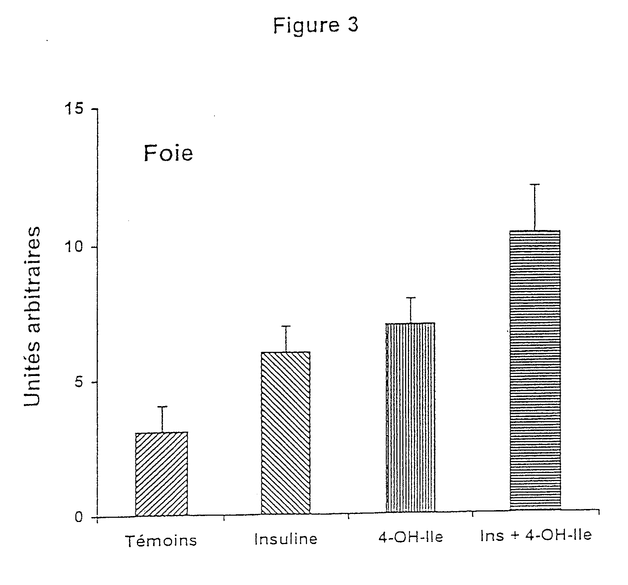

Les résultats en histogrammes sont donnés sur la figure 3. Sur ce schéma, on observe que l'insuline mais aussi la 4-OH-Ile, seules, stimulent significativement (p < 0,05) l'activité PI 3-kinase hépatique. Lorsque les deux substances sont administrées ensemble, un effet plus important apparaît.

Ces observations démontrent que la 4-OH-Ile possède des effets insulino-mimétiques au niveau du foie. De plus, l'effet plus important objectivé lorsque les deux substances sont injectées conjointement est en faveur de mécanismes d'activation synergiques.

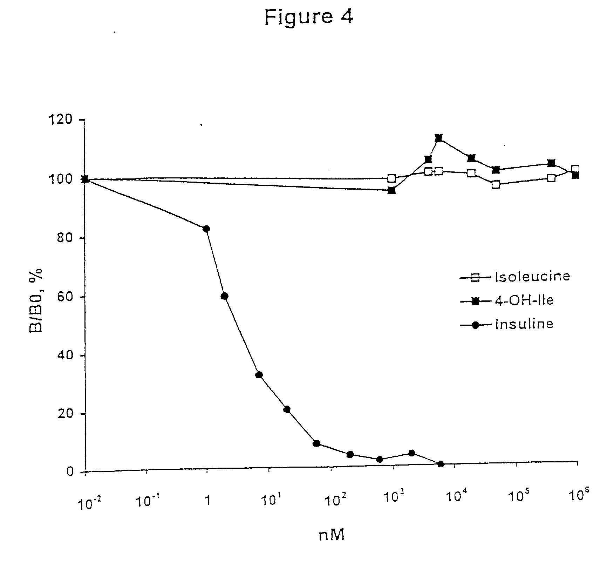

Ceci est confirmé par l'étude comparative des effets de l'insuline et de la 4-hydroxyisoleucine sur le récepteur membranaire à l'insuline de l'hépatocyte: la 4-hydroxyisoleucine n'a aucune liaison avec ce récepteur. On rapporte sur la figure 4 les résultats comparatifs concernant les liaisons de l'isoleucine et de la 4-OH-Ile. Les cellules LMH utilisées dans ces essais sont issues d'un hépatocarcinome de poulet (Kawaguchi T. et coll., Cancer Res., 47, 4460, 1987).

### EXEMPLE 3 : Comparaison des effets de l'insuline et de la 4-OH-Ile sur l'activité PI 3-kinase du muscle

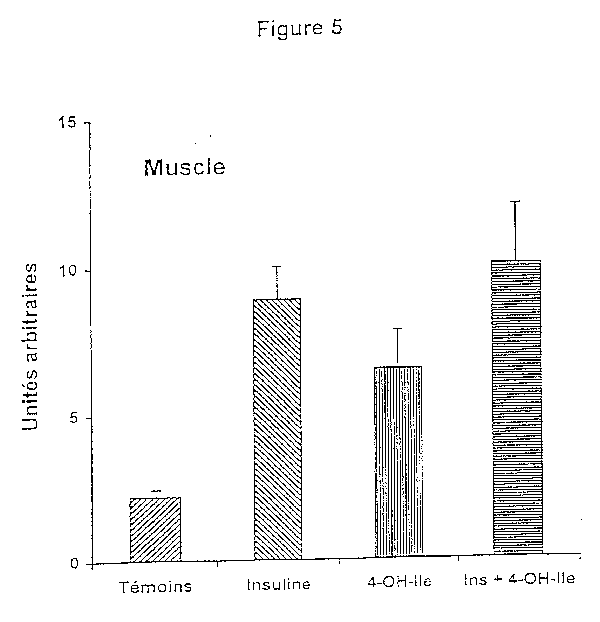

Les résultats en histogrammes sont reportés sur la figure 5. Sur ce schéma, on note que non seulement l'insuline stimule clairement l'activité PI 3-kinase, mais aussi que la 4-OH-Ile a un effet comparable. Ainsi, les effets insulino-mimétiques de la 4-OH-Ile observés au niveau hépatique sont confirmés au niveau musculaire.
On constate donc à l'examen des résultats des exemples 1 et 2 qu'au niveau du foie et du muscle, la 4-hydroxyisoleucine stimule indépendamment de l'insuline la voie IRS-1/PI 3-kinase qui est la voie majeure dans le contrôle des actions métaboliques et mitogéniques de l'insuline. De plus, la 4-hydroxyisoleucine ne reconnaît pas le récepteur de l'insuline ou du moins ne rentre pas en compétition avec l'insuline pour la liaison sur le récepteur.

### EXEMPLE 4

L'effet bénéfique de la 4-hydroxyisoleucine contre une relative hyperinsulinémie a été recherché *in vivo* lors de l'administration chronique (pendant un mois) de l'acide aminé végétal (25 mg/kg/jour, par voie intrapéritonéale) sur le rat rendu diabétique non insulino-dépendant (diabète de type 2) par les injections conjointes de nicotinamide et de streptozotocine (Masiello et coll., Diabetes, 47, 224, 1998). Des prises de sang régulières effectuées sur la veine caudale des rats ont permis d'évaluer par méthode radio-immunologique (Herbert et coll., J. Clin. Endocr., 25, 1375, 1965) le taux d'insuline plasmatique, 15 heures après l'injection du produit qui avait lieu à 17 h 30. Sur ces mêmes prélèvements, le taux plasmatique de glucose a été dosé par méthode enzymatique (Trinder P., J. Clin. Pathol. 22, 158, 1969).

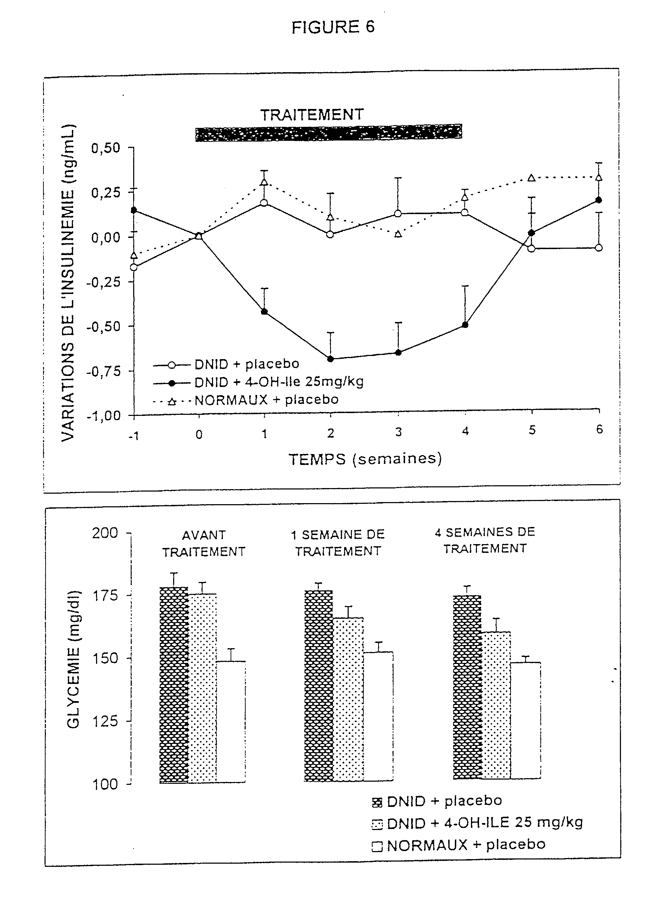

Les résultats obtenus sont rapportés sur la figure 6.

On observe que l'administration quotidienne de 4-hydroxyisoleucine a pour effet d'abaisser significativement (p < 0,05) l'insulinémie des animaux traités.

De plus, après l'arrêt du traitement, on peut constater que l'insuline plasmatique s'élève à nouveau pour rejoindre des valeurs proches de celles observées avant le traitement. Dans ces conditions, une légère diminution de la glycémie a été observée en fin de traitement.
L'abaissement de l'insulinémie observée chez le rat après un traitement chronique par la 4- hydroxyisoleucine confirme *in vivo* les effets insulino-mimétiques et/ou insulino-sensibilisateurs observés lors des expériences *in vitro.*

### EXEMPLE 5 : Comparaison des effets de l'insuline et de ceux de la 4-OH-Ile sur l'activité PI 3-kinase du muscle et du foie prélevés chez le rat diabétique de type 2 (Masiello et coll., Diabetes 47, 224, 1998)

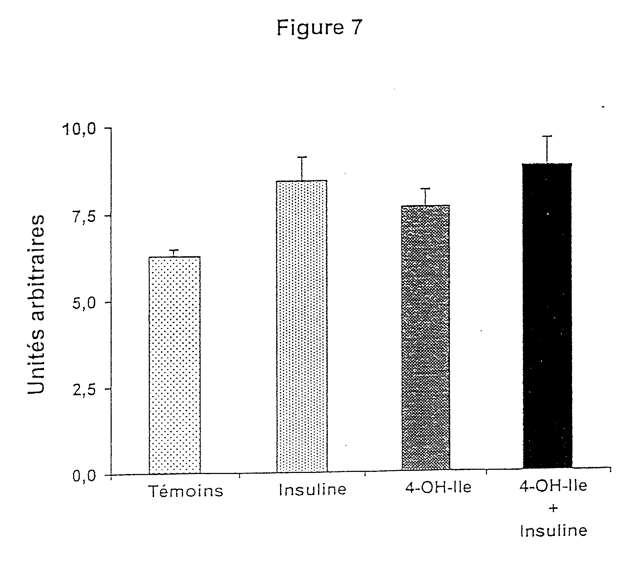

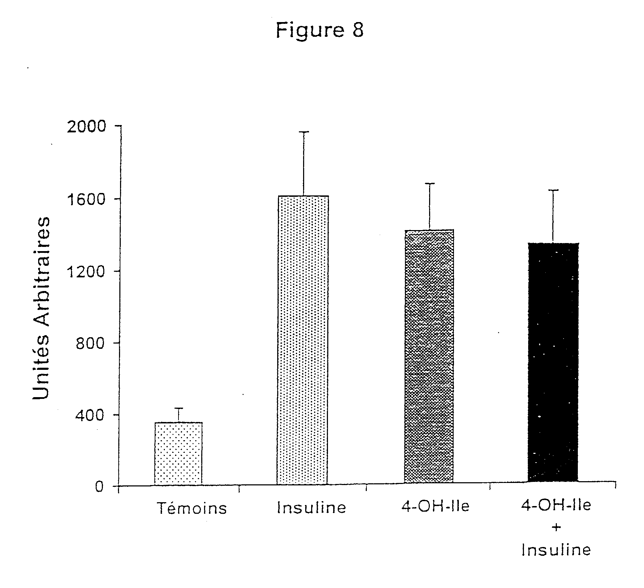

Les résultats en histogrammes sont reportés sur les figures 7 et 8. On note que, si l'insuline active la PI 3-kinase hépatique (figure 7) et musculaire (figure 8), il en est de même pour la 4-OH-Ile. La 4-OH-Ile dans cette situation pathologique induit le même effet que l'insuline avec la même intensité.
De plus, on constate que la 4-OH-Ile stimule significativement la voie IRS-1/PI 3-kinase plus nettement dans le muscle (p < 0,01) que dans le foie (p < 0,05).

### EXEMPLE 6

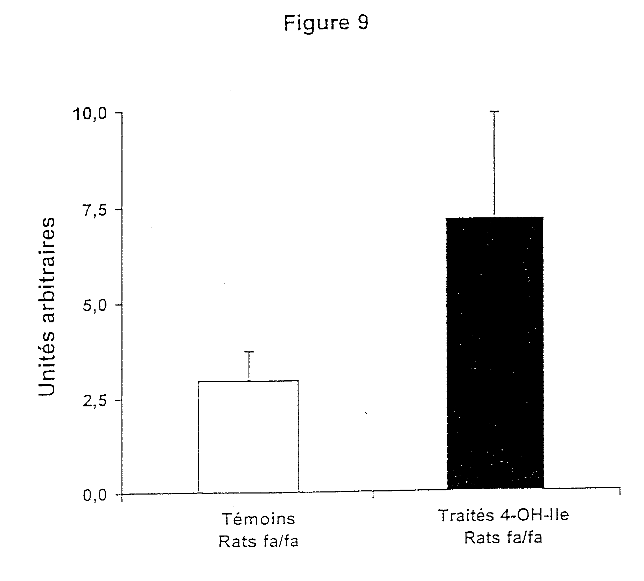

Lors de l'administration chronique pendant 4 semaines de la 4-OH-Ile (50 mg/kg par voie intrapéritonéale) chez le rat Zucker obèse *(fa*/*fa),* l'activité PI 3-kinase du foie a été mesurée en fin de traitement. Les résultats en histogrammes sont reportés sur la figure 9. Ils montrent que l'activité basale de la PI 3-kinase (animaux sacrifiés 17 heures après la dernière administration de 4-OH-Ile) est augmentée chez les animaux traités.

### EXEMPLE 7

La 4-OH-Ile active la PI 3-kinase associée au récepteur de l'insuline mais pas celle associée au récepteur PDGF (Platelet Derived Growth Factor).

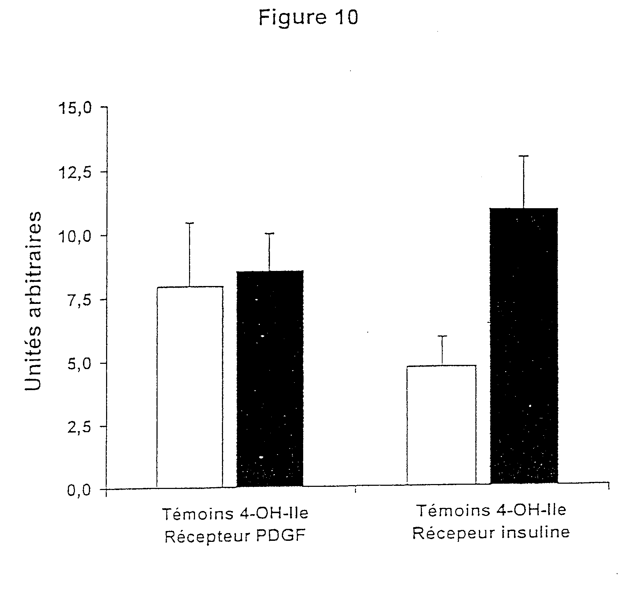

Les activités PI 3-kinase du récepteur PDGF et du récepteur-insuline en présence ou en l'absence de 4-OH-Ile (18 mg/kg I.P.) ont été comparées suivant le protocole déjà décrit.
Les résultats en histogrammes sont reportés sur la figure 10: ils démontrent clairement la spécificité de la voie d'action de la 4-OH-Ile au niveau du foie de rat, c'est-à-dire que l'activité de la PI 3-kinase associée au récepteur-insuline est la seule augmentée (p < 0,05).

### EXEMPLE 8 : Effet de la 4-OH-Ile sur l'activité phosphatase associée à la voie de signalisation du récepteur-insuline

Dans le but de mieux situer le site d'action de la 4-OH-Ile, son impact sur l'activité phosphatase a été étudié. L'activité phosphatase associée à IRS-1 a été mesurée.
La figure il montre que la 4-OH-Ile (200 µmol/l) inhibe significativement (p < 0,05) cette activité au niveau du foie de rat normal.

### EXEMPLE 9

Un médicament selon l'invention est particulièrement adapté au traitement symptomatique des états d'insulino-résistance, notamment des états d'insulino-résistance avec obésité. Il peut être préparé à partir d'un acide aminé mono- ou polyhydroxylé et/ou ses formes lactoniques à l'aide de toute technique appropriée connue de l'homme du métier. Il peut être notamment réalisé à partir de 4- hydroxyisoleucine. Ce produit étant hydrosoluble, un tel médicament peut être aisément réalisé sous forme de solution (dans du sérum physiologique par exemple), ou sous forme galénique solide telle que comprimé ou gélule. Les pathologies visées étant chroniques, l'administration par voie orale apparaît plus adaptée. De tels médicaments peuvent ainsi être aisément administrés à des posologies pluri-quotidiennes, adaptées au cas particulier du patient concerné, par exemple de l'ordre de 2 à 3 prises par jour. Un médicament selon l'invention peut également comprendre de l'insuline.

## Revendications

1. Utilisation d'un composé choisi parmi le groupe constitué par les acides aminés mono-hydroxylés, les acides aminés poly-hydroxylés, et les formes lactoniques de ces acides, pour la fabrication d'un médicament à effets insulino-mimétiques et/ou insulino-sensibilisateurs.

2. Utilisation selon la revendication 1, **caractérisée en ce que** ledit médicament est destiné à exercer un effet insulino-mimétique et/ou insulino-sensibilisateur au niveau des tissus périphériques cibles de l'insuline.

3. Utilisation selon la revendication 1 ou 2, **caractérisée en ce que** ledit composé diminue l'activité phosphatase associée à la voie de signalisation du récepteur de l'insuline, et/ou stimule l'activité PI 3-kinase sur IRS-1 et/ou IRS-2.

4. Utilisation selon l'une quelconque des revendications précédentes, **caractérisée en ce que** ledit composé est la 4-hydroxyisoleucine de formule 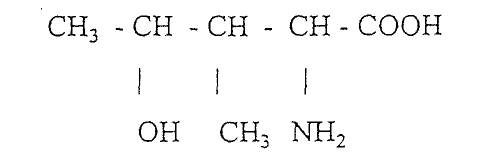 et/ou la forme lactonique de cet acide aminé.

5. Utilisation selon la revendication 4, **caractérisée en ce que** la 4-hydroxyisoleucine se présente sous la forme de son isomère 2S, 3R, 4S ou de la lactone correspondante.

6. Utilisation selon l'une quelconque des revendications 1 à 5, pour la fabrication d'un médicament destiné au traitement des insulino-résistances.

7. Utilisation selon l'une quelconque des revendications 1 à 6, pour la fabrication d'un médicament destiné à lutter contre les syndromes liés à l'insulino-résistance.

8. Utilisation selon l'une quelconque des revendications 1 à 7, pour la fabrication d'un médicament contre les hyperinsulinémies.

9. Utilisation selon l'une quelconque des revendications 1 à 7, pour la fabrication d'un médicament contre l'insulino-résistance liée au vieillissement.

10. Utilisation l'une quelconque des revendications 1 à 7, pour la fabrication d'un médicament contre les affections liées à l'obésité.

11. Utilisation selon l'une quelconque des revendications 1 à 7, pour la fabrication d'un médicament destiné à inhiber la prolifération de lignées cellulaires associées au risque d'apparition de cancers.

12. Utilisation selon l'une quelconque des revendications 1 à 5, pour la fabrication d'un médicament destiné à la prévention des insulino-résistances.

13. Utilisation selon l'une quelconque des revendications 1-5, et 12, pour la fabrication d'un médicament destiné à diminuer le besoin en insuline exogène.

14. Composition ou kit pharmaceutique comprenant à la fois de l'insuline et un composé choisi parmi le groupe constitué par les acides aminés mono-hydroxylés, les acides aminés poly-hydroxylés, et les formes lactoniques de ces acides.
